## Europäisches Patentamt

## European Patent Office

## Office européen des brevets

(11) Veröffentlichungsnummer: **0 647 131 B1**

# EUROPÄISCHE PATENTSCHRIFT

(45) Veröffentlichungstag der Patentschrift: **06.12.95**

(21) Anmeldenummer: **93912636.3**

(22) Anmeldetag: **24.06.93**

(86) Internationale Anmeldenummer:
**PCT/DE93/00572**

(87) Internationale Veröffentlichungsnummer:
**WO 94/00109 (06.01.94 94/02)**

(51) Int. Cl.⁶: **A61K  9/127**, A61K 31/02,
A61K 31/025, A61K 31/13,
A61K 31/34

(54) **DERMATIKUM ZUR UNTERSTÜTZUNG DES SAUERSTOFFTRANSPORTES IN DER HAUT.**

(30) Priorität: **26.06.92 DE 4221268**

(43) Veröffentlichungstag der Anmeldung:
**12.04.95 Patentblatt  95/15**

(45) Bekanntmachung des Hinweises auf die
Patenterteilung:
**06.12.95 Patentblatt  95/49**

(84) Benannte Vertragsstaaten:
**AT BE CH DE DK ES FR GB GR IE IT LI LU MC
NL PT SE**

(56) Entgegenhaltungen:
**WO-A-89/08459
WO-A-91/00110
WO-A-91/16068
WO-A-92/06676
DE-A- 4 127 442**

(73) Patentinhaber: **LANCASTER GROUP AG
Ludwig-Bertram Strasse 8-10
D-67059 Ludwigshafen (DE)**

(72) Erfinder: **GROSS, Udo
Falkenberger Chaussee 91
D-13059 Berlin (DE)**
Erfinder: **ZASTROW, Leonhard
Grabenweg 13
D-65205 Wiesbaden-Nordenstadt (DE)**
Erfinder: **RÖDING, Joachim
Trompeterstr. 19
D-65207 Wiesbaden-Rambach (DE)**
Erfinder: **STANZL, Klaus
Im Eschbach 9d
D-56323 Waldesch (DE)**

(74) Vertreter: **Walter, Wolf-Jürgen et al
Patentanwälte Felke & Walter
Normannenstrasse 1-2
D-10367 Berlin (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Dermatikum, das in den corialen Bereich der Haut sowie angrenzendes Gewebe penetriert und dabei eine verbesserte Sauerstoffversorgung in diesem Bereich erbringt.

In der US-A-4366169 (White) wird die Verwendung von Fluorcarbonen zur Behandlung von Hautverletzungen und Wunden, insbesondere von Verbrennungen, beansprucht. Dabei wird das sauerstoffenthaltende Fluorcarbon entweder direkt oder als Emulsion auf die Haut, auf entsprechende Verbände oder ähnliche Mittel gebracht. In der US-A-4569784 (Moore) wird die Herstellung eines Gels mit Gastransporteigenschaften zur Anwendung auf der Haut beschrieben. Das aufwendige Verfahren besteht darin, daß eine mit Wasser nicht mischbare organische Flüssigkeit, z. B. ein Fluorcarbon, in Gegenwart eines Emulgators durch ein Emulgierverfahren emulgiert wird. Es schließt sich ein Konzentrierungsprozeß an (z. B. Ultrazentrifugierung, Ultrafiltration), der zur Bildung einer Gel-Phase führt. In dem dann folgenden dritten Schritt wird die Trennung der klaren Flüssigkeit von dem pastösen Feststoff (Gel-Phase) durch Dekantieren, Filtrieren oder Verdampfen bewirkt. Dieses Gel wird in geeigneten Formulierungen auf der Haut angewendet, und es wirkt dort, ohne jedoch das Stratum corneum zu durchdringen.

In der EP-A-296661 (Borgarello) wird ein Fluorcarbon-haltiges Einphasensystem beschrieben, das als isotrope oder anisotrope Formulierung im kosmetischen Bereich und auch als Dermatikum als Sauerstofftransporteur wirken kann. Dabei werden Fluorcarbone mit einer maximalen Konzentration von 50 % mit perfluorierten Emulgatoren vom Alkansulfonsäureamid-Typ in Gegenwart eines aliphatischen Alkohols als Hilfsemulgator in Wasser emulgiert.

Die WO-A-89 08 459 beschreibt eine Perfluorcarbonemulsion mit Phospholipidvesikeln als Blutersatzstoff, bei der die Phospholipidmonomeren polymerisiert werden. In der WO-A-91 00110 werden Fluorcarbonemulsionen mit Phospholipiden offenbart, bei denen das Phospholipid gesättigte Kohlenstoffbindungen hat. Aus der WO-A-9206676 sind Öl-gefüllte wenig-lamellare Vesikel aus Phospholipiden bekannt, deren Struktur der üblichen Vesikelstruktur entspricht.

Bei den beschriebenen bekannten Verfahren wirken die beschriebenen Zusammensetzungen, die Fluorcarbone enthalten, systembedingt auf der Haut am Ort ihrer Aufbringung.

Der vorliegenden Erfindung liegt die Aufgabe zugrunde, das Stratum corneum der Haut und die Epidermis durch Penetrationsvorgänge zu überwinden, um im corialen Bereich und dem daran angrenzenden Gewebe die Sauerstoffkonzentration zu erhöhen und Stoffwechselvorgänge zu aktivieren.

Erfindungsgemäß besteht das Dermatikum zur Unterstützung des Sauerstofftransports in der Haut aus asymmetrischen lamellaren Aggregaten, die sich aus Phospholipiden mit einem Phosphatidylcholingehalt von 30 bis 99 Gew.-% aufbauen, die in ihrem Kern im Unterschied zu den gut bekannten wäßrigen Liposomen Fluorcarbone oder Gemische davon enthalten. Das erfindungsgemäße Dermatikum besteht somit aus asymmetrischen lamellaren Aggregaten, die aus Phospholipiden mit einem Phosphatidylcholingehalt von 30 bis 99 Gew.-% und mit sauerstoffbeladenem Fluorcarbon oder Fluorcarbongemisch bestehen, wobei der Anteil an Fluorcarbon im Bereich von 1 bis 100 % w/v (w/v = Gewicht/ Volumen) liegt, in einem für die dermatologische Anwendung geeigneten Trägerstoff.

Die lamellaren Aggregate sind wegen ihrer Phospholipidstruktur, die den Zellmembranen strukturell und chemisch sehr ähnlich ist bzw. zum Teil identisch ist, sowie in Verbindung mit ihrer bei der Herstellung steuerbaren Größe der Aggregate in der Lage, in tiefer liegende Hautschichten zu penetrieren und dort wirksam zu werden. Dies erfolgt im Gegensatz zu den im Stand der Technik genannten Erfindungsbeschreibungen, die nicht den Transport der Fluorcarbone in tieferliegende Regionen der Haut gestatten. Die bekannten Verfahren sind in Bezug auf den beanspruchten Effekt wirkungslos.

Es können eine Vielzahl von Fluorcarbonen eingesetzt werden, z.B. aliphatische geradkettige und verzweigte Fluoralkane, mono- oder bicyclische und gegebenenfalls fluoralkylsubstituierte Fluorcycloalkane, perfluorierte aliphatische oder bicyclische Amine, Bis-(perfluoralkyl)-Ethene oder deren Gemische. Besonders bevorzugt sind solche Fluorcarbone wie Perfluordecalin, F-Butyltetrahydrofuran, Perfluortributylamin, Perfluoroctylbromid, Bis-Fluor(Butyl)ethen oder Bis-Fluor(Hexyl)ethen oder $C_6$-$C_9$-Perfluoralkane.

Dabei liegt der Anteil an Fluorcarbonen im Bereich von 1 bis 100 % w/v, vorzugsweise im Bereich von 40 bis 100 %. Ein besonders bevorzugter Bereich ist der von 70 bis 100 % w/v.

Als Phospholipide werden erfindungsgemäß natürliche Phospholipide wie Sojalecithin und Eilecithin sowie synthetische Phospholipide eingesetzt. Bei diesen Phospholipiden liegt der Gehalt an Phosphatidylcholin im Bereich von 30 bis 99 Gew.-%, insbesondere 70 bis 99 Gew.-%, d.h. Phospholipide mit hohen Phosphatidylgehalten sind bevorzugt.

Neben Phosphatidylcholin können auch Lysolecithine im Konzentrationsbereich von 0,1 bis 10 Gew.-% und/oder geladene Phospholipide wie Phosphatidylethanolamin, n-Acetylphosphatidylethanolamin oder Phosphatidsäure im Konzentrationsbereich 0,1 bis 30 Gew.-% vorhanden sein.

Im Unterschied zu den bekannten wäßrigen Liposomen (Vesikel) tragen die erfindungsgemäßen Phospholipid-stabilisierten Aggregate in ihrem Kern hydrophobe Fluorcarbone, die zum Transport von Sauerstoff befähigt sind. Ihre grenzflächenchemische Stabilisierung erfolgt primär durch eine Monolayer mit inverser Anordnung und sekundär durch einen sich daran anschließenden Aufbau von Bilayer- Schichten. Erfindungsgemäß weisen die asymmetrischen lamellaren Aggregate daher zumindest immer eine dreischichtige Struktur auf im Unterschied zu den zweischichtigen bekannten Vesikeln. Wegen der Besonderheit ihrer strukturellen Anordnung werden diese neuartigen Aggregate als asymmetrische lamellare Sauerstoff-Carrier bezeichnet. Ihre außergewöhnliche kolloidchemische Stabilität ist vermutlich auf die lamellare Struktur und auf die Oberflächenladung der Aggregate zurückzuführen. Letztere ist auf die Auswahl geeigneter Phospholipide beziehungsweise deren Mischungen natürlicher wie auch synthetischer Provenienz zurückzuführen. In erster Linie sind für eine vorteilhafte Wirkung in diesem Sinne Phospholipide, insbesondere Phosphatidylcholin im genannten Konzentrationsbereich von 30 bis 99 % in Verbindung gegebenenfalls mit Lysolecithinen der Konzentration von 0,1 bis 10 % und/oder geladenen Phopsholipiden im Konzentrationsbereich 0,1 bis 30 Gew.-% verantwortlich. Die angesprochene Wirkung der Phospholipide wird durch entsprechende negative Zeta-Potentiale und durch die Messung von Ladungsdichten (bei Titration mit einem kationischen Polyelektrolyten) verifiziert.

Die Abhängigkeit der Penetrationsgeschwindigkeit und der Eindringtiefe von der Teilchengröße der Aggregate konnte durch separate Untersuchungen im Tierexperiment mit markierten verkapselten Fluorcarbonen experimentell bestimmt werden. Danach wandern kleinere Teilchen schneller und tiefer in das Hautgewebe als größere Partikel. Die Auswahl von Fluorcarbonen bzw. deren Mischungen nach ihrer Lipidlöslichkeit (dargestellt durch ihre kritische Löslichkeitstemperatur CST in n-Hexan) erlaubt als ein weiteres wichtiges Kriterium die Regelung der Verweilzeit im Gewebe. Während z. B. Perfluortributylamin (F-TBA, CST 59° C) mit einem hohen CST-Wert und schlechter Lipidlöslichkeit eine größere Verweilzeit aufweist, wird im Gegensatz dazu Perfluordecalin (PFD, CST 22° C) aber auch F-Butyltetrahydrofuran, F-Hexan und andere entsprechend schneller aus dem Gewebe abgegeben. Mit Hilfe von Fluorcarbonmischungen lassen sich auf diese Weise gezielt Systeme mit gewünschten CST-Werten d. h. Lipid- und Membranlöslichkeiten bezüglich der beabsichtigten Anwendung herstellen.

Der Gehalt der Fluorcarbone als Sauerstoffträger in den lamellaren Aggregaten kann entsprechend dem Anwendungzweck zwischen 1 und 100 % w/v variieren. Als Fluorcarbone kommen insbesondere in Betracht:

aliphatische geradkettige und Verzweigte Alkane mit 6 bis 12 Kohlenstoffatomen, wie z. B. Perfluorhexan, Perfluornonan;

mono- oder bicyclische Cycloalkane, die gegebenenfalls F-alkylsubstituiert sind, wie z. B. Perfluormethylcyclohexan, Perfluordecalin;

aliphatische tertiäre Amine, N-haltige Polycyclen, wie z. B. Perfluortripropylamin, Perfluortributylamin;

Perfluorether, wie aliphatische Ether und Polyether, F-alkyl-Furane, bicyclische und substituierte bicyclische Ether mit 2 oder 3 Sauerstoffatomen im Molekül, wie z. B. Perfluordihexylether, Perfluorbutyltetrahydrofuran;

Perfluoralkylhalogenide, wie z. B. Perfluoroctylbromid, Perfluorhexylbromid, Perfluoroctylchlorid;

Bis-F(Alkyl)ethene, wie z. B. Bis-F(Butyl)ethen, Bis-F(Hexyl)ethen.

Unter dem hier verwendeten Begriff "Fluorcarbone" werden perfluorierte oder hochfluorierte Kohlenstoffverbindungen oder Gemische verstanden, die in der Lage sind, Gase wie $O_2$ und $CO_2$ zu transportieren. Hochfluorierte Kohlenwasserstoffverbindungen sind im Sinne dieser Erfindung solche, bei denen die meisten Wasserstoffatome durch Fluoratome ersetzt sind, wie z.B. die Bis-F(Alkyl)ethene, die nachweislich chemisch und biologisch inert und damit untoxisch sind. Dies wird meist dann erreicht, wenn etwa bis zu 90 % der Wasserstoffatome durch Fluoratome ersetzt sind. Bevorzugt im Sinne der vorliegenden Erfindung sind Fluorcarbone, bei denen wenigstens 95 % der Wasserstoffatome ersetzt sind, bevorzugter 98 % und am bevorzugtesten 100 %.

Es können auch einzelne Fluoratome durch andere Halogenatome wie Brom oder Chlor ersetzt sein.

Als Phospholipide kommen natürlich auftretende Phospholipide wie Soja- oder Eilecithin in Frage, sowie auch synthetisch herstellbare Lecithine (Phospholipide), die insgesamt als hautfreundlich bekannt sind. Wegen der vorteilhaften Wirkung auf die Stabilität der asymmetrischen lamellaren Aggregate kommen vorzugsweise Phospholipidmischungen mit einem Anteil von 30 bis 99 % an Phosphatidylcholin neben weiteren natürlich auftretenden Begleitprodukten zur Verwendung. Der Phospholipidgehalt in der dermatologischen Formulierung bewegt sich zwischen 0,5 und 20, vorzugsweise 10 bis 20 %.

Die Erfindung betrifft auch ein Verfahren zur Herstellung eines Phospholipide enthaltenden Dermatikums, das darin besteht, daß Phospholipide mit einem Phosphatidylcholingehalt von 30 bis 99 Gew.-% emulgiert werden mit einem Fluorcarbon oder einem Fluorcarbongemisch, welches mit Sauerstoff beladen ist, wobei der Anteil an Fluorcarbon im Bereich von 0,2 bis 100 % w/v liegt, und die dabei erhaltenen asymmetrischen lamellaren Aggregate mit einer Teilchengröße von 50 bis 1000 nm in einem für die dermatologische Anwendung geeigneten Träger eingearbeitet werden. Die Emulgierung erfolgt in Anwesenheit von Wasser und gegebenenfalls unter Zusatz von einwertigen oder mehrwertigen aliphatischen Alkoholen. Die Emulgierung kann auch erfolgen durch eine Voremulgierung der Rohdispersion durch Zugabe des Fluorcarbons zu einer wäßrigen Phospholipidlösung bei einer Temperatur entsprechend den eingesetzten Ausgangsstoffen. Die Voremulgierung erfolgt zweckdienlich bei höheren Umdrehungszahlen, z. B. 12000 bis 15000 U/min. Die eigentliche Homogenisierung erfolgt dann in einem Hochdruckhomogenisator. Die Homogenisierung kann auch mit anderen bekannten Verfahren wie zum Beispiel Ultraschall erfolgen. Der Grad des Energieeintrags in das disperse System wirkt sich indirekt proportional auf die Teilchengrößen aus.

Eine Hitzesterilisierung im Autoklaven ist ohne eine Beeinflussung der Teilchengrößen möglich. Zur Vermeidung von Autoxydationsprozessen im ungesättigten Fettsäurerest nativer Lipide können Antioxydantien, z. B. $\alpha$-Tocopherol zugesetzt werden.

Besonders vorteilhaft ist der Einsatz von Phospholipiden mit hohen Phospatidylcholingehalten. Diese liegen im allgemeinen zwischen 10 und 99 Gew.-%, Vorzugsweise 30 bis 99 Gew.-%, insbesondere 70 bis 99 Gew.-%.

Als Fluorcarbone werden die oben genannten Fluorcarbone bzw. Fluorcarbongemische verwendet in den dafür angegebenen Grenzen. Mit Hilfe von deren bekannten $O_2$-Löslichkeiten, des Dampfdrucks und der kritischen Lösichkeitstemperatur kann vom Fachmann gezielt die Beladung mit Sauerstoff und die Eindringtiefe eingestellt werden.

Die Wirkungsweise der Fluorcarbon-haltigen asymmetrischen lamellaren Aggregate beruht auf der Abgabe von Sauerstoff an unterversorgtes Gewebe über eine topische Applikation. Eine sinnvolle Anwendung ist auch denkbar für Sauerstoff-unterversorgtes Fettgewebe wie auch für arteriosklerotisch bedingte Mangelversorgungen.

Die Einarbeitung der asymmetrischen lamellaren Aggregate als wirksame Substanz in Salben, Cremes, Lotionen und anderen wäßrigen oder alkoholischen dermatologischen Formulierungen erfolgt in Abhängigkeit Vom Anwendungszweck, wobei der Fluorcarbongehalt und damit die $O_2$-Verfügbarkeit in breiten Grenzen variiert werden kann. Die Aggregate können vor der Einarbeitung in alle dermatologischen Systeme wie z.B. Gele, Pasten, Puder, Salben, Cremes, Lotionen mit gasförmigem Sauerstoff partiell beladen beziehungsweise gesättigt werden. Bereits die Sättigung mit dem Sauerstoff der atmosphärischen Luft durch die üblicherweise stattfindende Gleichgewichtseinstellung entsprechend dem Henry'schen Gesetz bietet eine höhere Sauerstoffkapazität als alle vergleichbaren bekannten Systeme.

Das erfindungsgemäße Dermatikum kann auch auf Verbände, Pflaster, Wundabdeckungen, und sonstige mit der Haut in Berührung kommende Mittel aufgebracht werden. Es kann beispielsweise auch als Spray appliziert werden.

Die Erfindung soll nachstehend durch Beispiele näher erläutert werden. In der dazugehörigen Zeichnung bedeuten

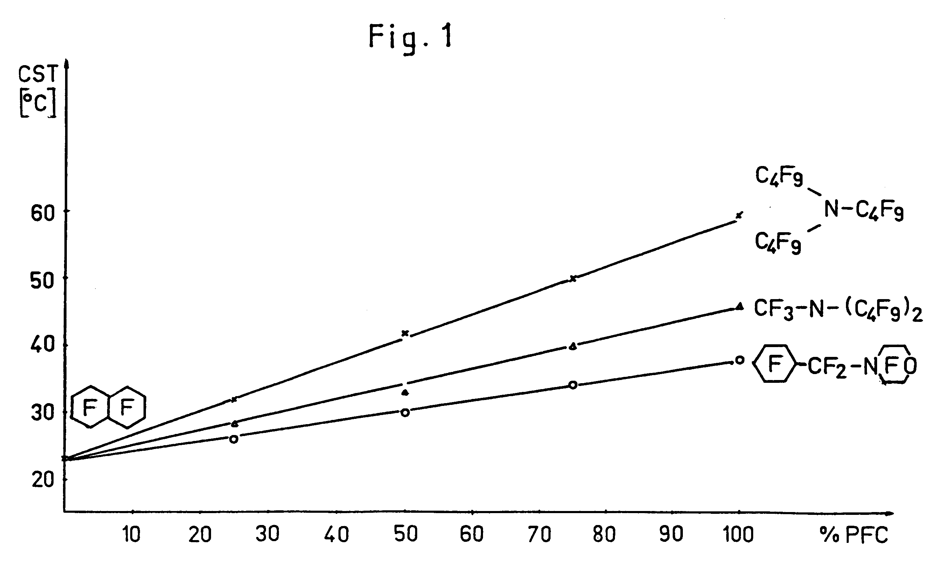

Fig.1 Diagramm der kritischen Löslichkeitstemperaturen (CST) von Perfluorcarbongemischen in n-Hexan mit Perfluordecalin als Ausgangspunkt

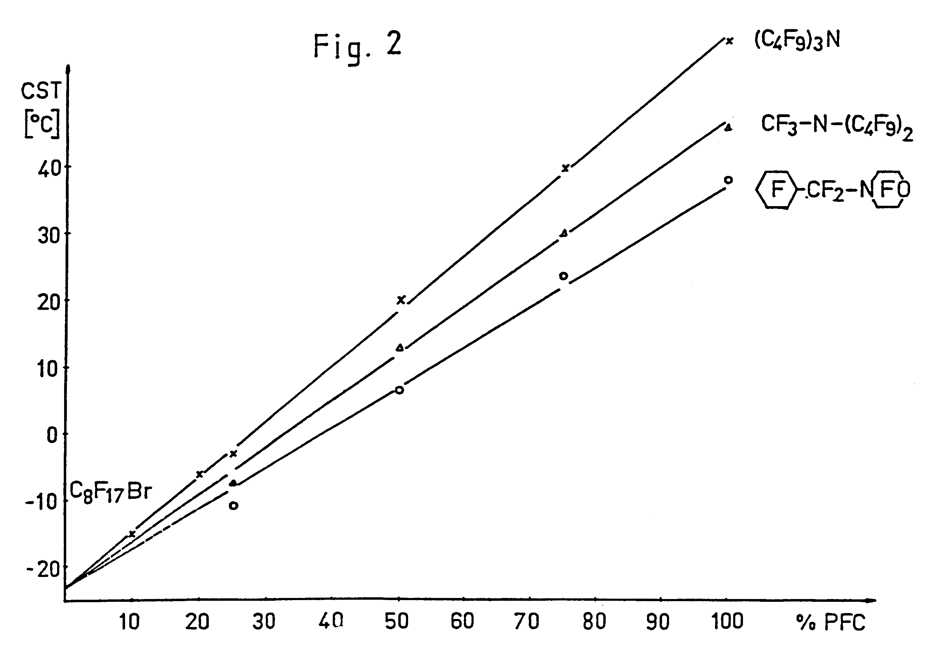

Fig.2 Diagramm der kritischen Löslichkeitstemperaturen von Perfluorcarbongemischen in n-Hexan mit F-Octylbromid als Ausgangspunkt.

In Tabelle 1 sind einige ausgewählte Fluorcarbone und ihre $O_2$-Löslichkeit, ihr Dampfdruck und die kritische Löslichkeitstemperatur dargestellt. Ausgehend von diesen Werten können für Gemische von Fluorcarbonen die gewünschten Charakteristika bei der Penetrierung der Haut mit Hilfe einer dermatologischen Zusammensetzung ausgewählt werden.

4

Tabelle 1

| Fluorcarbon | $O_2$-Löslichkeit [ml $O_2$/100 ml FC] | Dampfdruck $P_{37\,°C}$ [mm Hg] | CST [°C] |
|---|---|---|---|
| Perfluoroctylbromid | 50 | 14 | -24,5 |
| Perfluordecalin | 40 | 12,5 | 22 |
| Bis-F(Butyl)ethen | 50 | 12,6 | 22,5 |
| F-cyclohexylmethylmorpholin | 42 | 4 | 38,5 |
| F-Tripropylamin | 45 | 18,5 | 43 |
| F-Dihexylether | 45 | 2 | 59 |
| F-Tributylamin | 40 | 1 | 59 |
| Perfluordecalin-F-Tributyl-amin 1/1 | 40 | 7 | 42 |
| Perfluorbutyltetrahydrofuran | 52 | 51 | 29 |
| F-methylcyclohexan | 57 | 180 | 8,2 |
| F-Hexan | 58 | 414 | 20 |

Beispiel 1

50 ml einer 10%igen wäßrigen Phospholipidlösung (Sojalecithin, 40 % Phosphatidylcholin (PC)) werden gemeinsam mit 80 g eines hochreinen, keine H-Atome enthaltenden Fluorcarbongemisches (90 % Perfluordecalin, 10 % F-Dibutylmethylamin, kritische Löslichkeitstemperatur 26 °C) mit einem Ultraschalldesintegrator unter Eiskühlung homogenisiert bis die Teilchen einen mittleren Durchmesser von 244 nm aufweisen. Aus $^{31}$P-NMR-Messungen ist anhand der typischen Signalbreite wie auch aus elektronenmikroskopischen Aufnahmen die lamellare Struktur der Aggregate aus Fluorcarbon und Phospholipid zu erkennen. Die Aggregatdispersion läßt sich unproblematisch und ohne Beeinflussung ihrer Stabilität mit geeigneten Alkoholen (Ethanol, Propylenglycol, Glycerol) zum Zweck der Sterilisierung mischen. Ein Zusatz von 30 ml Ethanol erzeugt Keimfreiheit, wobei die resultierende Dispersion folgende Zusammensetzung aufweist:

62 % w/v Fluorcarbone

9,7 % Phospholipide

19 % Ethanol

Das Zeta-Potential von minus 61 mV belegt eine durch die Phospholipide erzeugte negative Oberflächenladung mit einer elektrostatischen Stabilisierung der Dispersion. Nach Sättigung mit gasförmigem Sauerstoff wird die Dispersion in eine mit den asymmetrischen lamellaren Aggregaten verträgliche, nicht wechselwirkende Salbengrundlage eingearbeitet.

Beispiel 2

18 g lyophilisiertes Phospholipid der Zusammensetzung [30 % PC, 30 % PE (Phosphatidylethanolamin), 31 % PI (Phosphatidylinositol)] werden in 90 ml sterilisiertem Wasser gelöst und mit 16 ml unvergälltem Ethanol versetzt. Mit einem mechanischen hochtourigen Rührer (Ultra-Turrax, 15 000 U/min) wird die Dispersion gerührt und dabei nacheinander Perfluordecalin (CST 22 °C) dem auf 20 °C temperierten Rührbehälter zugesetzt. Die Rohdispersion wird in einem Hochdruckhomogenisator vom Typ Manton Gaulin bei 500 atm im Inertgasstrom homogenisiert. Mit Beginn des vorletzten Durchlaufs werden der Dispersion $\alpha$-Tocopherolacetat zur Vermeidung von Autoxidationsprozessen und als Fänger für freie Radikale im Hautgewebe zu 0,1 % zugesetzt.

Die mit dem Photonenkorrelationsspektrometer N-4 MD (Coultronics) durchgeführten Messungen bestätigen das Vorliegen einer unimodalen Teilchengrößenverteilung und einen mittleren Teilchendurchmesser von 128 nm. Elektronenmikroskopische Untersuchung bei Anwendung der Methode des "negative staining" sind in Übereinstimmung damit. Nach $^{31}$P-NMR-Untersuchungen liegen die asymmetrischen lamellaren Aggregate im unilamellaren Zustand vor mit einem Zeta-Potential von minus 76 mV. Die Zusammensetzung der Dispersion beträgt

48 % w/v Perfluordecalin

13 % Phospholipide

9 % Ethanol.

Beispiel 3

80 g n-F-Hexan, das im Gemisch mit seinen perfluorierten Isomeren vorliegt (CST 20 °C) wurden mit 9,5 Gramm Eigelb 3-sn Phosphatidylcholin in 47 ml deionisiertem und sterilisiertem Wasser unter Inertgasbedingungen bei Zusatz von 0,2 % dl-Alpha-Tocopherol zu einer Rohdispersion mechanisch voremulgiert. Die Rohemulsion wurde im Druckhomogenisator bei Drücken von 500 atm bei einem geeigneten Temperaturregime und unter Kontrolle der Teilchengrößen homogenisiert. Die erhaltene Dispersion weist eine mittlere Viskosität und einen Teilchendurchmesser von 294 nm auf. Nach Zusatz von 8 ml Propylenglycol wurde im Langzeitversuch Stabilität und Keimfreiheit (Keimzahl kleiner als 100 K/g) bei Raumtemperatur beobachtet. Eine Verdünnung, z.B. bei der Herstellung von Lotionen, ist ohne eine Änderung wichtiger kolloidchemischer Parameter problemlos möglich.
Untersuchungen der Dispersion mit dem Lichtmikroskop im polarisierten Licht zeigen das Vorliegen eines isotropen Einphasensystems an, in dem flüssig-kristalline Strukturen nicht existent sind.

Beispiel 4

In vivo Nachweis der Liposomen-Penetration

Eine frisch isolierte physiologisch intakte Haut wurde mit ihrer Innenseite auf einen $O_2$-Sensor (Clark-Elektrode) fixiert und die Epidermis mit einer $O_2$-transportierenden Dispersion mit asymmetrischen lamellaren Aggregaten benetzt. Unter diesen Bedingungen zeigt die Elektrode keinen $O_2$-Partialdruck an. Nach 57 Minuten Penetrationsdauer hatten die Aggregate den dermalen Hautabschnitt im Meßbereich der Elektrode erreicht. Der $O_2$-Partialdruck stieg auf einen Wert von 159 mm Hg an. Die Penetrationsgeschwindigkeit in der Haut ist abhängig von Art und Größe der Aggregate.

**Patentansprüche**

1. Dermatikum zur Unterstützung des Sauerstofftransportes in der Haut, gekennzeichnet durch asymmetrische lamellare Aggregate, bestehend aus
   (a) Phospholipiden mit einem Phosphatidylcholingehalt von 30 bis 99 Gew.-%, und
   (b) mit Sauerstoff beladenem Fluorcarbon oder Fluorcarbongemisch, wobei dar Anteil an Fluorcarbon im Bereich von 0,2 bis 100 % Gewicht/Volumen liegt,
   wobei die Aggregate eine Hautpenetrierung in Abhängigkeit von der kritischen Löslichkeitstemperatur (in n-Hexan) der ausgewählten Fluorcarbone oder Fluorcarbongemische haben, und in einem für die dermatologische Anwendung geeigneten Träger vorliegen.

2. Dermatikum nach Anspruch 1, dadurch gekennzeichnet, daß die lamellaren Aggregate von deren Fluorcarbon-Kern ausgehend eine asymmetrische, vorzugsweise dreischichtige Struktur aufweisen.

3. Dermatikum nach Anspruch 1 oder 2, dadurch gekennzeichnet, daß die Fluorcarbone aus der Gruppe ausgewählt sind, die aus aliphatischen geradkettigen und verzweigten Fluoralkanen, mono-oder bicyclischen gegebenenfalls fluoralkylsubstituierten Fluorcycloalkanen, perfluorierten aliphatischen oder bicyclischen Aminen, Bis-(perfluoralkyl)-ethenen oder deren Gemischen besteht.

4. Dermatikum nach Anspruch 3, dadurch gekennzeichnet, daß die Fluorcarbone aus der Gruppe ausgewählt sind, die aus Perfluordecalin, F-Butyltetrahydrofuran, Perfluortributylamin, Perfluoroctylbromid, Bis-Fluor(butyl)ethen oder $C_6$-$C_9$-Perfluoralkanen besteht.

5. Dermatikum nach einem der Ansprüche 1 bis 4, dadurch gekennzeichnet, daß der Anteil an Fluorcarbonen im Bereich von 20 bis 100 % Gewicht/Volumen liegt, vorzugsweise im Bereich von 40 bis 100 %, insbesondere im Bereich von 70 bis 100 %.

6. Dermatikum nach einem der Ansprüche 1 bis 5, dadurch gekennzeichnet, daß die Phospholipide ausgewählt sind aus der Gruppe, bestehend aus natürlichen Phospholipiden wie Sojalecithin und Eilecithin sowie synthetischen Phospholipiden und/oder teilhydrierten Phospholipiden, wobei die Konzentration der Phospholipide im Bereich von 0,5 bis 20 % liegt.

7. Dermatikum nach einem der Ansprüche 1 bis 6, dadurch gekennzeichnet, daß Phosphatidylcholin in einem Anteil von 60 bis 90 % vorhanden ist.

8. Dermatikum nach einem der Ansprüche 1 bis 7, dadurch gekennzeichnet, daß die verwendete Lipidfraktion neben Phosphatidylcholin Lysolecithine im Konzentrationsbereich von 0,1 bis 5 Gew.-% enthält.

9. Verfahren zur Herstellung eines Dermatikums zur Unterstützung des Sauerstofftransportes in der Haut, dadurch gekennzeichnet, daß Phospholipide mit einem Phosphatidylcholingehalt von 30 bis 99 Gew.-% emulgiert werden mit einem mit Sauerstoff beladenen Fluorcarbon oder Fluorcarbongemisch, wobei der Anteil an Fluorcarbon im Bereich von 0,2 bis 100 % Gewicht/Volumen liegt, und die dabei erhaltenen asymmetrischen lamellaren Aggregate mit einer Teilchengröße von 50 bis 1000 nm in einen für die dermatologische Anwendung geeigneten Träger eingearbeitet werden.

10. Verfahren nach Anspruch 9, dadurch gekennzeichnet, daß der Anteil an Fluorcarbonen im Bereich von 20 bis 100 % Gewicht pro Volumen liegt, vorzugsweise im Bereich von 40 bis 100 % und der Anteil an Phosphatidylcholin in der verwendeten Lipidfraktion im Bereich von 60 bis 90 % liegt.

11. Verfahren nach Anspruch 9 oder 10, dadurch gekennzeichnet, daß die Teilchengröße im Bereich von 120 bis 820 nm liegt, Vorzugsweise zwischen 140 und 400 nm.

12. Verwendung zur Herstellung eines Dermatikums zur Steuerung der Sauerstoffversorgung der Haut durch Auftragen eines Systems mit einem asymmetrischen lamellaren Sauerstoff-Carrier, enthaltend Phospholipide mit einem Phosphatidylcholingehalt von 30 bis 99 Gew.-% und Fluorcarbone, wobei die Fluorcarbone im Bereich von 0,2 bis 100 % Gewicht/Volumen liegen und wobei die Penetration in die Haut über die Carrier-Struktur der Phopholipid-Aggregate und die kritische Löslichkeitstemperatur der Fluorcarbone (in n-Hexan) gesteuert wird, und das System in einem für die dermatologische Anwendung üblichen Träger wie Salben, Cremes, Lotionen, Wässer, alkoholische Auszüge, Pasten, Puder, Gele, Tinkturen verteilt vorliegt oder das System auf einem Verband oder einem Pflaster aufgetragen ist oder mittels eines Sprays appliziert wird.

**Claims**

1. Dermatological agent for assisting the transport of oxygen in the skin, characterised by asymmetric lamellar aggregates, consisting of
   (a) phospholipids having a phosphatidylcholine content of 30 to 99 % by weight, and
   (b) oxygen-laden fluorocarbon or fluorocarbon mixture, the amount of fluorocarbon being in the range from 0.2 to 100 % weight/volume,
   the aggregates having a skin penetration depending on the critical solubility temperature (in n-hexane) of the selected fluorocarbons or fluorocarbon mixtures, and being present in a carrier which is suitable for dermatological use.

2. Dermatological agent according to Claim 1, characterised in that the lamellar aggregates have an asymmetric, preferably three-layer, structure originating from their fluorocarbon core.

3. Dermatological agent according to Claim 1 or 2, characterised in that the fluorocarbons are selected from the group which consists of aliphatic straight-chain and branched fluoroalkanes, mono- or bicyclic, optionally fluoroalkyl-substituted, fluorocycloalkanes, perfluorinated aliphatic or bicyclic amines, bis-(perfluoroalkyl)ethenes and mixtures thereof.

4. Dermatological agent according to Claim 3, characterised in that the fluorocarbons are selected from the group which consists of perfluorodecalin, F-butyltetrahydrofuran, perfluorotributylamine, perfluorooctyl bromide, bis-fluoro(butyl)ethene and $C_6$-$C_9$-perfluoroalkanes.

5. Dermatological agent according to one of Claims 1 to 4, characterised in that the amount of fluorocarbons is in the range from 20 to 100 % weight/volume, preferably in the range from 40 to 100 %, in particular in the range from 70 to 100 % weight/volume.

6. Dermatological agent according to Claim 1, characterised in that the phospholipids are selected from the group consisting of natural phospholipids such as soya lecithin and egg lecithin, synthetic phospholipids, hydrogenated lecithins and/or partially hydrogenated phospholipids, the concentration of the phospholipids being in the range from 0.5 to 20 %.

7. Dermatological agent according to Claim 1, characterised in that phosphatidylcholine is present in an amount from 60 to 90 %.

8. Dermatological agent according to Claim 1, characterised in that in the lipid fraction used, in addition to phosphatidylcholine, lysolecithins are present in the concentration range from 0.1 to 5 % by weight.

9. Process for the preparation of a dermatological agent for assisting the transport of oxygen in the skin, characterised in that phospholipids having a phosphatidylcholine content of 30 to 99 % by weight are emulsified with an oxygen-laden fluorocarbon or fluorocarbon mixture, the amount of fluorocarbon being in the range from 0.2 to 100 % weight/volume, and the asymmetric lamellar aggregates having a particle size from 50 to 1000 nm obtained in this way are incorporated into a carrier which is suitable for dermatological use.

10. Process according to Claim 9, characterised in that the amount of fluorocarbons is in the range from 20 to 100 % weight per volume, preferably in the range from 40 to 100 % and the amount of phosphatidylcholine in the lipid fraction used is in the range from 60 to 90 %.

11. Process according to Claim 9, characterised in that the particle size is in the range from 120 to 820 nm, preferably between 140 and 400 nm.

12. Use for production of a dermatological agent for controlling the supply of oxygen to the skin by application of a system containing an asymmetric lamellar oxygen carrier, containing phospholipids having a phosphatidylcholine content of 30 to 99 % by weight and fluorocarbons, the fluorocarbons being in the range from 0.2 to 100 % weight/volume and the penetration into the skin being controlled by means of the carrier structure of the phospholipid aggregates and the critical solubility temperature of the fluorocarbons (in n-hexane), and the system being present distributed in a carrier which is customary for dermatological use, such as ointments, creams, lotions, waters, alcoholic extracts, pastes, powder, gels and tinctures or the system being applied to a dressing or a plaster or being applied by means of a spray.

**Revendications**

1. Produit de dermatologie ou pour traitement d'une dermatose, destiné à renforcer le transport de l'oxygène dans la peau, produit caractérisé par la présence d'agrégats lamellaires asymétriques, consistant en :
   a) des phospholipides ayant une teneur en de la phosphatidyl choline de 30 à 99 % en poids, et
   b) du fluorocarbone ou un mélange de fluorocarbones chargé(s) d'oxygène, la proportion du fluorocarbone se situant entre 0,2 et 100 % en poids/volume
   les agrégats ayant une pénétration dans la peau qui dépend de la température de solubilité critique (dans du n-hexane) des fluorocarbones ou des mélanges de fluorocarbones choisis, et étant présents dans un support convenant pour une application dermatologique.

2. Produit pour utilisation en dermatologie ou pour traitement d'une dermatose selon la revendication 1, caractérisé en ce que les agrégats lamellaires présentent une structure asymétrique, avantageusement en trois couches, qui dérive de leur noyau fluorocarboné.

3. Produit de dermatologie ou pour traitement d'une dermatose selon la revendication 1 ou 2, caractérisé en ce que les fluorocarbones sont choisis dans l'ensemble consistant en des fluoroalcanes aliphatiques linéaires et ramifiés, des fluorocycloalcanes monocycliques ou bicycliques éventuellement substitués par un ou, des groupes fluoroalkyles, des amines perfluorées aliphatiques ou bicycliques, des bis-(perfluoroalkyl)-éthènes ou leurs mélanges.

4. Produit de dermatologie ou pour traitement d'une dermatose selon la revendication 3, caractérisé en ce que les fluorocarbones sont choisis dans l'ensemble consistant en de la perfluorodécaline, du F-butyltétrahydrofuranne, de la perfluorotributyl amine, du bromure de perfluoro-octyle, du bis-trifluoro-(butyl)éthène ou des perfluoro-alcanes en $C_6$ à $C_9$.

5. Produit pour dermatologie ou pour traitement d'une dermatose selon l'une des revendications 1 à 4, caractérisé en ce que la proportion des fluorocarbones se situe entre 20 et 100 % en poids/volume, avantageusement entre 40 et 100 %, notamment entre 70 et 100 %.

6. Produit pour dermatologie ou pour traitement d'une dermatose selon l'une des revendications 1 à 5, caractérisé en ce que les phospholipides sont choisis dans l'ensemble consistant en des phospholipides naturels comme de la lécithine de soja et de la lécithine d'oeuf, ainsi que parmi des phospholipides synthétiques et/ou des phospholipides partiellement hydrogénés, la concentration des phospholipides se situant entre 0,5 et 20 %.

7. Produit pour dermatologie ou pour traitement d'une dermatose selon l'une des revendications 1 à 6, caractérisé en ce que la phosphatidyl choline est présente en une proportion de 60 à 90 %.

8. Produit pour dermatologie ou pour traitement d'une dermatose selon l'une des revendications 1 à 7, caractérisé en ce que la fraction lipidique utilisée contient, en plus de la phosphatidyl choline, des lysolécithines présentes en une concentration de 0,1 à 5 % en poids.

9. Procédé pour préparer un produit pour dermatologie ou pour traitement d'une dermatose, pour soigner et renforcer le transport de l'oxygène dans la peau, produit caractérisé en ce que l'on émulsifie des phospholipides, ayant une teneur en phosphatidyl choline de 30 à 90 % en poids, avec un fluorocarbone ou un mélange de fluorocarbones chargé(s) d'oxygène, la proportion du fluorocarbone se situant entre 0,2 et 100 % en poids/volume, et l'on incorpore les agrégats lamellaires asymétriques ainsi obtenus, ayant une grosseur particulaire de 50 à 1000 nm, dans un support convenant pour l'application en dermatologie.

10. Procédé selon la revendication 9, caractérisé en ce que la proportion des fluorocarbones se situe entre 20 et 100 % en poids par volume, avantageusement entre 40 et 100 %, et en ce que la proportion de la phosphatidyl choline dans la fraction lipidique utilisée se situe entre 60 et 90 %.

11. Procédé selon la revendication 9 ou 10, caractérisé en ce que la grosseur des particules se situe entre 120 et 820 nm, avantageusement entre 140 et 400 nm.

12. Utilisation, pour préparer un produit pour dermatologie ou pour traitement d'une dermatose, en vue de réguler l'apport en oxygène de la peau, par application d'un système comportant un support d'oxygène, lamellaire et asymétrique, contenant des phospholipides ayant une teneur en phosphatidyl choline de 30 à 99 % en poids et contenant des fluorocarbones, les fluorocarbones étant présents en une proportion de 0,2 à 100 % poids / volume, et la pénétration dans la peau étant régie par la structure du support des agrégats phospholipidiques et par la température de solubilité critique des fluorocarbones (dans le n-hexane), et le système étant présent en répartition dans un support usuel pour servir en dermatologie comme des baumes ou pommades, des crèmes, des lotions, des eaux, des extraits alcooliques, des pâtes, de la poudre, des gels, des teintures ou bien le système étant placé sur une bande de support ou sur un emplâtre ou bien étant appliqué à l'aide d'une projection par nébulisation ou atomisation ("spray").

Fig. 1

EP 0 647 131 B1

Fig. 2

EP 0 647 131 B1